# EUROPEAN PATENT SPECIFICATION

(11) **EP 3 810 106 B1**
(45) Date of publication and mention of the grant of the patent: **01.11.2023**
(21) Application number: 19748947.9
(22) Date of filing: 21.06.2019
(51) Int. Cl.: A61K 31/065, A61K 31/198, A61K 31/216, A61K 31/365, A61K 31/7048, A61K 33/06, A61K 36/19, A61K 9/20, A61P 27/16, A61P 43/00

(54) **COMPOSITION COMPRISING N-ACETYLCYSTEINE AND ANDROGRAPHOLIDE FOR USE IN THE PREVENTION AND/OR TREATMENT OF SENSORINEURAL HEARING LOSS OF THE INNER EAR**
ZUSAMMENSETZUNG AUS N-ACETYLCYSTEIN UND ANDROGRAPHOLID ZUR VORBEUGUNG ODER BEHANDLUNG VON SENSORINEURALEM HÖRVERLUST IM INNENOHR
COMBINAISON DU N-ACÉTYL-CYSTÉINE ET DE L'ANDROGRAPHOLIDE DESTINÉE À LA PRÉVENTION OÙ AU TRAITEMENT DE LA PERTE AUDITIVE NEUROSENSORIELLE DE L'OREILLE INTERNE

(30) Priority: 21.06.2018 IT 201800006566
(43) Date of publication of application: 28.04.2021
(73) Proprietor: ALERE VITAM S.R.L., 51034 Serravalle Pistoiese (IT)
(72) Inventor: SCAPAGNINI, Giovanni, 95131 Catania (IT); PAVONE, Vito, 95127 Catania (IT); DAVINELLI, Sergio, 86100 Campobasso (IT); WILLCOX, Donald Graig, Urasoe City Okinawa Prefecture, 901-2111 (JP)
(74) Representative: Valenza, Silvia
(86) International application number: PCT/IB2019/055249
(87) International publication number: WO 2019/244121

(56) References cited:
- EP-A1- 2 845 589
- WO-A1-2016/191468
- CN-A- 1 663 605
- US-A1- 2003 044 512
- US-A1- 2003 191 064
- JUN LIU ET AL: "In Vivo and In Vitro Anti-inflammatory Activities of Neoandrographolide", THE AMERICAN JOURNAL OF CHINESE MEDICINE, vol. 35, no. 02, 1 January 2007 (2007-01-01), pages 317-328, XP055558007, US ISSN: 0192-415X, DOI: 10.1142/S0192415X07004849

## Description

The present invention concerns a composition for preventing or treating sensorineural hearing loss of the inner ear.

### PRIOR ART

Hearing loss, commonly referred to as deafness, is a disorder of the auditory area that presents with reduced or compromised hearing. Hearing loss can be caused by damage due to the degeneration of one or more of the anatomical components that form the auditory organ.

Generally, hearing loss is classified both on the basis of the site of the auditory damage and on the basis of the extent of the deficiency.

On the basis of the extent of the acoustic deficiency it is possible to catalogue the hearing loss into four classes.

A first class is represented by mild hearing loss, characterized by a reduction of the hearing threshold between 20 and 40 dB. The second class comprises moderate-mild hearing loss wherein the auditory deficiency varies within the range from 40 to 65 dB. Severe hearing loss is the third class into which subjects with an auditory deficiency comprised between 65 and 85 dB are classified. The last class into which subjects with a deficiency greater than 85 dB are classified is deafness.

On the basis of the cause of the auditory deficiency it is instead possible to divide the hearing loss into different types.

A first type is defined as transmissive hearing loss. This presents when the damage is localized in the area of the outer ear or in the transmissive structures of the middle ear. In these cases the acoustic deficiency is generally moderate, usually less than 50-60 decibel.

The main causes of transmissive hearing loss include those of a physical type, such as traumatic ones or those due to the presence of foreign bodies in the ear, or those that result from diseases such as otitis, herpes, exostosis and simple ear drum perforations.

Sensorineural hearing loss is caused by damage localized in the cochlea or in the acoustic nerve and is a pathology that can affect both young subjects and adults. In the latter case it can have a rapid course and in these cases we talk about sudden deafness, autoimmune based hearing loss and fluctuating or slow onset hearing loss such as in cases of chronic acoustic trauma or presbycusis (ARHL) and acoustic neuroma.

Hearing loss can also be of the mixed type when the auditory damage affects both the external transmission functions of the sound wave such as the outer and middle ear and the transduction apparatus represented by the cochlea and the transmission apparatus, comprising the acoustic nerve.

The most frequent cause of hearing loss is however connected with the ageing of the anatomical structures that form the auditory apparatus, a phenomenon also known as presbycusis.

According to the most recent data of the World Health Organization (WHO), hearing loss currently represents the third disability in the world population after arterial hypertension and joint diseases.

It emerges from scientific data how hearing loss has been increasing exponentially in the population from the age of sixty onwards.

For example, Van Eyken et al in Audiology and Neurotology 2007; 12(6):345-58 document how mild hearing loss problems, i.e. with hearing loss greater than 25 dB, affect about 37% of the adult population aged between 61 and 70. This percentage reaches 60% in the population aged between 71 and 80, even reaching 80% in subjects over 80.

There are indications for establishing a prevention or treatment action for hearing loss as soon as the first symptoms of acoustic deficiency present.

The type of treatment is selected as a function of the type and gravity of the symptoms and the age of the individual.

However, it has been found that in forms of hearing loss connected with the advancement of age, subjects with acoustic deficiency tend to delay the start of treatments that can slow down the progression of the auditory deficiency.

Most of the population in the advanced age only intervene on the problem when the acoustic deficiency is mild or moderate-mild, a condition in which there are not many effective remedies available.

This situation can partly be attributed to the absence or reduced availability of products able to intervene at the start of the acoustic deficiency, exercising a recovery action of the physiological conditions of the auditory apparatus.

From a nutritional point of view, the initial forms of acoustic deficiency are generally treated by resorting to the integration of the diet with products that usually boast an antioxidant action on the cells but which in most cases do not have a specific action on the organs involved in the auditory function.

However there are currently very few products with a nutritional or therapeutic action that are indicated for preventing or treating sensorineural hearing loss, in particular that can be attributed to senescence.

US 2003/044512 discloses compositions comprising *Andrographis paniculata* for use in the treatment of ear oedema.

Am. J. Chin. Med. 2007, 35, 317 discloses compositions comprising *Andrographis paniculata* for use in the treatment of otitis media.

US 2003/191064 discloses compositions comprising *N*-acetylcysteine for use in the treatment of vestibular disorders.

WO 2016/191468 discloses compositions comprising *N*-acetylcysteine for use in the treatment of otological disorders.

An object of the present invention consists of providing a composition for preventing or treating sensorineural hearing loss of the inner ear.

### SUMMARY OF THE INVENTION

The inventors have found that by administering a combination of N-acetylcysteine and androgpraholide, a synergistic action is obtained stimulating specific cell structures of the auditory apparatus determining an improvement of the auditory functions and a slowing down in the progression of the decline of the auditory functions in the treated subject.

According to a first aspect, a composition is therefore provided comprising N-acetylcysteine (also referred to herein as NAC) and andrographolide and an edible vehicle for use in the prevention and/or treatment of sensorineural hearing loss of the inner ear.

Further embodiments of the composition of the invention are defined in the claims.

According to some embodiments, the andrographolide is in the form of a plant extract, in particular a plant extract of Andrographis paniculata.

In accordance with certain embodiments of the invention, the inventors have further identified that the synergistic action of andrographolide and N-acetylcysteine is increased by rosmarinic acid, a substance provided with cellular antioxidant activity.

Typically in the composition according to the invention the active ingredients andrographolide and N-acetylcysteine are provided in a pharmaceutically, nutraceutically or dietetically effective quantity.

According to some embodiments, the composition of the invention is a pharmaceutical composition, a nutraceutical, or a dietary supplement that can be introduced into the dietary regime of an individual affected by sensorineural hearing loss of the inner ear.

The present invention will be described in detail below making reference to the following figures.

### BRIEF DESCRIPTION OF THE DRAWINGS

Some characteristics and advantages of the present invention will become clear from the appended figures in which:
Figure 1 illustrates a bar type graph showing the results of an MTT assay on hair cells of the organ of Corti with the aim of evaluating the toxicity of a composition containing a combination of andrographolide and N-acetylcysteine with respect to the control;
Figure 2 shows how treatment with HEI-OC1 cells with the combination of andrographolide and N-acetylcysteine activates and surprisingly increases the expression of Nrf2 antioxidant transcription factor;
Figure 3 illustrates through bar graphs the results of an MTT assay performed to evaluate cell vitality following cytotoxicity induced by cisplatin, highlighting how combined NAC + andrographolide treatment has cytoprotective effects;
Figure 4 illustrates through bar graphs the results of an apoptotic oligonucleosomal detection method of the "sandwich enzyme-linked immunosorbent assay" type. The results highlight how treatment with the Andrographolides [Andr.] + N-acetylcysteine [NAC] combination significantly reduced the apoptosis rate in cells treated with cisplatin [Cisp.].

### DETAILED DESCRIPTION OF THE INVENTION

The inventors have found that the combination of N-acetylcysteine and andrographolide acts on the Nef2 system, which represents the elected biological target for preventing and/or treating hearing loss, induced by noise. It has also been observed how the use of N-acetylcysteine and andrographolide in combination determines the reduction of the oxidative damage of the cochlea.

From these experimental observations, the inventors have formulated a composition provided with otoprotective properties, useful for restoring sensorineural hearing loss of the inner ear in a mammal, in particular a human being.

It has also been found that taking the combination of N-acetylcysteine and a andrographolide in an effective quantity, performs a protective action against oxidative stress of the hair cells outside the organ of Corti improving the auditory capacities of the treated individual.

In accordance with a first aspect of the present invention a composition is therefore provided comprising N-acetylcysteine (also referred to herein as NAC) and andrographolide and an edible vehicle for use in the prevention and/or treatment of sensorineural hearing loss of the inner ear.

The activity and effectiveness of the composition of the invention in treating sensorineural hearing loss of the inner ear was demonstrated experimentally by testing the composition on an experimental in vitro model based on the oto-cytotoxicity of cisplatin. This model is widely used in the sector of the invention for miming the mechanisms associated with hearing loss. The results found that certify the therapeutic efficacy of the composition are reported in Example 3 below.

N-acetylcysteine or NAC represents an active ingredient in the synergistic composition of the invention. Chemically, NAC is an *N*-acetylate derivative of the amino acid sulfurated cysteine.

The integration into the diet with acetylcysteine reduces the levels of 8-hydroxydeoxyguanosine, an indicator of oxidative damage and frequency of DNA deletions. Furthermore, N-acetylcysteine is a precursor of Glutathione, a substance that reduces the formation of free radicals and of reactive oxygen species (ROS).

The surprising aspect however consists of the fact that by combining NAC with andrographolide or with an extract of Andrographis paniculata, a plant rich in diterpenoid lactones, a synergistic action of the Nrf2 system is obtained, which constitutes a biological target used in the experimental area as a predictive model of an activation activity of the auditory system of mammals.

According to some embodiments, the NAC present in the composition of the invention has a titer greater than 55%, greater than 95%, greater than 98%.

In preferred embodiments, the NAC used in the composition is highly pure and has an almost total absence of pollutants such as allergens, heavy metals, additives and preservatives, e.g. each of these contaminants may be present in a quantity less than or equal to 0.001%.

The NAC used in the formulation of the composition of the invention may be of any origin and may be obtained through conventional production processes. According to some embodiments, the composition of the invention contains N-acetylcysteine in an amount comprised from 50 to 5000mg, from 100 to 2000mg, from 250 to 750 mg.

According to some embodiments the composition of the invention contains N-acetylcysteine in an amount comprised from 0.1 to 95% by weight, from 1 to 90% by weight, from 10 to 85%, from 30 to 80% by weight.

The composition of the invention further comprises andrographolide or a plant extract or a portion or tissue of a plant containing andrographolide.

In the formulation of the composition it is possible to use andrographolide of plant or synthetic origin, however within the scope of the invention the use of andrographolide of plant origin is preferred, in particular obtained through extraction from a plant or portions thereof that contain it.

In some embodiments, the andrographolide is contained or extracted from Andrographis paniculata, a herbaceous plant of the Acanthaceae family. In particular, the extract is obtained by extraction from Andrographis paniculata leaves since they are rich in terpenoid lactones for use in the composition of the invention. In some embodiments, the andrographolide is contained or supplied in the form of a plant extract of a plant containing andrographolide.

Andrographolide has the following structure (a).

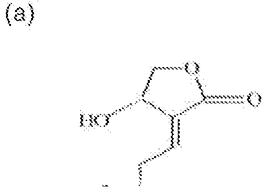

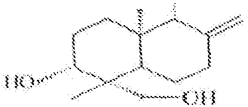

Andrographolide is an unsaturated trihydroxy lactone with molecular formula C₂₀H₃₀O₅.

The andrographolide of plant origin used in the composition according to the present invention can be obtained through a conventional solid-liquid extraction technique of the bioactive components from a portion of a plant containing them, such as for example Andrographis paniculata.

The extraction techniques that can be used to obtain said bioactive diterpenoid lactones are of the conventional type.

In the extraction of the andrographolide, it is possible to use suitable solvents, e.g. polar organic solvents such as ethanol.

For example Soxhlet extraction is a technique suitable for extracting and separating andrographolide from a plant source.

For example, the extraction of a sample of Andrographis paniculata can be performed by placing the sample in contact with a polar organic solvent, e.g. ethyl alcohol, optionally increasing the temperature of the system to reach a range from 30 to 70°C so that the heat applied to the distillation flask reaches the extraction cavity.

It is not usually necessary to perform any filtration after the leaching step. In some embodiments, the production of the sample can be increased through parallel simultaneous extraction.

A suitable solid-liquid extraction of andrographolide is described by R. Wongkittipong et al. in Solid-liquid extraction of Andrographolide from Plants-Experimental Study, Kinetic Reaction and Model; Sepn. Purifn. Technol. 40:147-154.

In some embodiments the aforesaid andrographolide or plant extract, portion or tissue of a plant containing such andrographolide can be present in the composition of the invention in an amount from 0.0001 to 35% by weight, from 0.001 to 20% by weight, from 0.1 to 10% by weight.

Andrographolide, neo-andrografolide and deoxyandrographolide are the most represented natural diterpenoid lactones isolated from A. paniculata. All three molecules are bioactive and in scientific literature they are defined as analogues in particular due to the biological activity; furthermore, all three exhibit similar activity in experimental *in vitro* and *in vivo* models of inflammation or cancer such as those described for example by Mishra SK, Tripathi S, Shukla A, Kim HM, "Andrographolide and analogues in cancer prevention" in Front Biosci (Elite Ed). 2015 Jan 1;7:255-66 and Lim JC1, Chan TK, Ng DS, Sagineedu SR, Stanslas J, Wong WSClin Exp Pharmacol Physiol "Andrographolide and its analogues: versatile bioactive molecules for combating inflammation and cancer" in Clin Exp Pharmacol Physiol. 2012 Mar; 39(3):300-10.

Andrographolide is part of the compositions described herein.

The inventors, using an experimental model comprising outer hair cells (OHCs), found that a mixture of NAC with one or more of the aforesaid andrographolide, as such or in the form of plant extracts, inhibits the pathway of the nuclear factor-kappa B (Nf-kb), which is pro-inflammatory, and the activation of the antiinflammatory pathway Nrf2.

By exercising such actions, the composition of the invention is effective in the prevention and treatment of auditory disorders such as hearing loss in particular sensorineural and in reducing oxidative-dependent stress cochlea damage. This activity is particularly clear for auditory disorders in which there is an inflammatory component and/or sensorineural hearing loss or perceptive hearing loss.

Due to its activities, the composition of the invention can also be applied in the treatment of vestibular disorders. This effect is closely connected with the effect on pathological ear disorders as the auditory system (peripheral and central) performs a significant role in balance control.

In mammals, hair cells act as sensory receptors both for the auditory system and for the vestibular system.

The vestibular toxicity of cisplatin has been ascertained in recent decades by various authors. Cisplatin damages sensory hair cells and the neurons in the cochlear and vestibular system. (Black et al., 1982; Kobayashi et al., 1987; Takimoto et al., 2016; Callejo et al., 20107; Ding et al., 2018). Different cell models and auditory and vestibular disorders have been developed, similar to our model and to the experimental conditions (So et al., 2005; Kim et al., 2016; Lee et al., 2005; Ma et al., 2016; Guo et al., 2018).

Cell lines have considerable potential in many areas of research into the auditory and vestibular apparatus. However, direct research into the molecular mechanisms of hearing loss and vestibular disorders is frequently limited by the small amount of tissue available for experimentation. Organotypic cochlear and vestibular cultures are not easy to obtain. Cell growth in culture may be an alternative approach; however, cochlear hair cells are terminally differentiated and do not proliferate *in vivo* or *in vitro.* This limitation was avoided by means of the development of different cell lines of the auditory apparatus through conditional immortalization (Rivolta et al., 1998, Rivolta et al., 2006; Kalinec et al., 1999).

Research into the auditory and vestibular system has benefited from the cell lines developed from different tissues and the phases of development of the inner ear. For the compositions of the invention, the NAC and andrographolide combination is conveniently formulated as a composition in which one or more edible or physiologically acceptable vehicles are present.

According to certain embodiments the composition of the invention contains andrographolide in an amount comprised from 000.1 to 1000mg, from 5 to 500, from 20 to 100mg.

Typically the basic active ingredients of the composition of the invention perform synergistic activity in preventing and/or treating sensorineural hearing loss of the inner ear.

In this application, the term "synergy or synergistic activity" means an activity which is greater than the sum of the activities of the individual active ingredient. In particular, the synergy occurs when at least two substances interact in a way in which one reinforces or increases one or more of the effects thereof. Consequently, two substances that produce similar effects sometimes produce increased effects when used simultaneously and a quantitative assessment is necessary for distinguishing these cases from a simple additive action (Tallarida RJ. Drug Synergism: its detection and applications. J Pharmacol. Exp. Ther. 2001 Sept.: 298(3):865-72).

According to some embodiments the composition of the invention further comprises rosmarinic acid.

Rosmarinic acid is chemically the ester between caffeic acid and the alcohol group in the α position of 3,4-dihydroxyphenylactic acid. Rosmarinic acid is a polyphenolic acid present in many plants belonging to the Lamiaceae family such as rosemary, sage.

Furthermore, the inventors have observed that the addition of rosmarinic acid on the NAC and andrographolide combination determines an unexpected increase of the activation of the Nrf2 and its main antioxidant enzyme HO-1.

This synergistic biological activity determines an unexpected increase in the treatment/prevention of sensorineural hearing loss

According to another embodiment the composition of the invention further comprises lutein, a carotenoid with high antioxidant properties. Lutein is a pigment that constitutes the retina necessary for the physiological functioning of the eye.

It has been observed that the addition of lutein to any of the embodiments of the composition previously described contributes to contrasting the sensorineural decline associated with the ageing of the human body involving the auditory system.

According to another embodiment, the composition of the invention further contains magnesium. It has been observed that its presence within the synergistic formulation of the invention contributes to protecting the nerves of the inner ear and contributes to increasing the antioxidant activity of NAC and andrographolide.

Furthermore, the presence of magnesium in the formulation of the composition of the invention contributes to maintaining the physiological level of magnesium in the inner ear fluid thus contributing to limiting the risks of auditory damage consequent to exposure to intense noise.

The amount of active compound in the compositions of the invention is such that a preventive and therapeutically effective dose for sensorineural hearing loss of the inner ear can be obtained.

In some embodiments, the composition of the invention can comprise one or more further substances or active ingredients.

In some embodiments, the composition of the invention further comprises one or more vitamins for example B group vitamins, niacin, vitamin A, vitamin C, vitamin PP, B group vitamins being preferred.

According to some embodiments, the composition of the invention further comprises one or more micronutrients and/or minerals such as Mg, K, Na, Zn, Fe, Cr, Se, Mn salts and others.

The term "vehicle" as used herein indicates a medium, excipient, diluent with which the association of therapeutic or active ingredients is administered.

Any vehicle and/or excipient suitable for the desired form of preparation for administration to human beings is contemplated for use with the compounds described in the present invention.

For the purposes of the present application, the term "edible" intends to indicate edible substances that are approved by the health authorities for use and/or in the formulation of phytotherapeutic, nutritional or dietary compositions.

The term "pharmaceutically acceptable" identifies a substance that can be used within the area of pharmaceutical formulations. A physiologically acceptable vehicle may be a pharmaceutically acceptable vehicle.

Within the scope of the present application the term combination means that one or more of the active ingredients are added or mixed with one or other ingredients. The term combination is not intended to mean that the active ingredients are associated with each other with the formation of chemical or other types of bonds. The compositions of the invention are suitable for dietary, nutritional, dietetic or pharmaceutical use in mammals, in particular in human beings.

The composition of the invention can assume a wide variety of forms of preparation, according to the desired route of administration.

The composition of the invention may be in solid form.

When the composition of the invention is presented in solid form it may be in the form of a tablet, capsule, powder, granules.

The preparations in solid form can comprise one or more vehicles/carriers such as for example amides, sugars, microcrystalline cellulose, and optionally diluents, granulation agents, lubricants, ligands, disintegration agents.

Typically the tablets, pills, capsules and granules can also contain a ligand such as tragacanth, acacia, corn starch or gelatin; excipients such as dicalcium phosphate; a disintegration agent such as corn starch, potato starch, alginic acid; a lubricant such as magnesium stearate; a sweetening agent such as sucrose, lactose or saccharin. If desired, the tablets can be coated using traditional techniques. When the pharmaceutical unit form is a capsule, it can contain in addition to the materials of the aforesaid type a liquid carrier such as a fatty oil.

According to certain embodiments the composition of the invention contains an excipient based on cellulose that comprises i) organic esters of cellulose for example selected from cellulose acetate, cellulose propionate, cellulose triacetate, cellulose acetate propionate, cellulose acetate butryate, ii) inorganic esters of cellulose for example selected from nitrocellulose, cellulose sulfate, iii) ethers of cellulose selected from a) cellulose alkyl ethers for example selected from methyl cellulose, ethyl cellulose, ethyl methyl cellulose; b) hydroxyalkyl ethers of cellulose for example selected from hydroxyethyl cellulose, hydroxypropyl cellulose, hydroxyethyl cellulose, hydroxypropyl methyl cellulose, ethyl hydroxyethyl cellulose; c) carboxyalkyl cellulose for example carboxy methyl cellulose, salts thereof and mixtures thereof. In certain embodiments the excipients based on cellulose are crosslinked with physiologically acceptable crosslinking agents.

In the composition there may also be flavouring agents, preservatives, colouring agents and the like.

In certain embodiments the composition of the invention is in solid tablet form. According to preferred embodiments, the composition of the invention is in the form of gastro-resistant tablets.

According to certain embodiments the composition is in a form for the modulated or controlled release of active ingredients. By way of example, the composition may be a delayed release, quick release or slow-fast release formulation. Typically, modulated or controlled release formulations contain one or more excipients based on cellulose, in particular of the type previously described.

In certain embodiments, the composition of the invention comprises as an excipient a hydrogenated fatty acid, preferably having a chain with 3 to 20 carbon atoms, 14 to 18 carbon atoms. A typical example of a hydrogenated fatty acid is hydrogenated palm oil.

The composition of the invention may be in liquid form.

When the composition is in liquid form, it may be in the form of a suspension, emulsion, solution. In these cases the vehicle is liquid and can be selected for example from water, glycols, oils, alcohol and mixtures thereof.

In certain embodiments in which the formulation is in liquid form, e.g. in cases of a syrup or elixir it can contain in addition to the association of active ingredients, saccharose as a sweetening agent, methyl- and propyl-parabens as preservatives, a colouring agent and a flavouring such as cherry or orange flavouring.

In some embodiments, the active ingredients contained in the composition of the present invention may be combined or mixed as active ingredients in intimate mixture with a suitable edible vehicle and/or an excipient according to the pharmaceutical and food industry or traditional nutritional techniques.

The compositions may be suitably presented in a single pharmaceutical form and prepared using any of the methods well known in the pharmaceutical or dietary state of the art.

In some embodiments, the composition of the invention further comprises one or more additional components such as additives, fillers, stabilizers, emulsifiers, texturizers, film-forming agents, plasticizers, wetting agents and thickeners. Various other materials may be present as coatings or to modify the physical shape of the pharmaceutical unit. For example, the tablets can be coated with shellac, sugar or both. To prevent the breakdown during transit through the upper part of the gastrointestinal tract, the composition may be a formulation with a gastro-resistant or enteric coating.

In some embodiments, in the compositions of the present invention, the active ingredients are normally formulated in a dosage unit. The dosage unit can contain from 0.1 to 1,000 mg of each active ingredient per dosage unit for daily administration.

In some embodiments, the formulation will contain quantities of active ingredients that depend on the gravity of the form of cognitive impairment and the related symptoms, the condition, other therapies being taken, the individual state of health and the response to the association of active ingredients.

In some embodiments the dose is in the interval from 0.001% by weight to about 60% by weight of the formulation.

According to some embodiments the composition of the invention is a medicinal product.

According to some embodiments, the composition of the invention is a nutraceutical, food supplement, a nutritional product or a dietetic product.

In accordance with another aspect, the invention relates to a composition comprising a combination of NAC and andrographolide and a physiologically acceptable vehicle and/or excipient for use in the prevention or treatment of sensorineural hearing loss of the inner ear.

Typically, the compositions for use according to the invention described herein have otoprotective/curative action and can be applied in the prevention and/or treatment of sensorineural hearing loss of the inner ear.

In particular, the composition described herein can be applied in the treatment of very mild hearing loss with a lowering of the auditory threshold that varies from 5 to 19 dB, mild hearing loss with a lowering of the auditory threshold that varies from 20 to 40 dB, moderate hearing loss with a lowering of the auditory threshold that varies from 41 to 65 dB, severe hearing loss that varies from 66 to 85 dB.

According to some embodiments, the composition for use comprises the administration of a quantity of NAC comprised between 50 and 1500 mg, typically from 100 to 1000 mg, in combination with andrographolide in a quantity from 10 to 250 mg, typically from 20 to 100 mg once or more times a day, typically once a day.

The composition of the invention is for use in the prevention or treatment of auditory disorders such as for example mild, moderate-mild, moderate to high hearing loss, in sensorineural hearing loss the inner ear.

The following examples are mainly provided to illustrate the present invention.

### EXAMPLE 1

Composition in the form of tablets for the prevention and treatment of the having the following formulation:

| | |
|---|---|
| N-acetylcysteine | 500 mg |
| Andrographolide | 50 mg |
| Excipients | 12 mg |

### EXAMPLE 2

Composition in the form of tablets for the treatment of sensorineural hearing loss having the following formulation:

| | |
|---|---|
| N-acetylcysteine | 500 mg |
| Andrographolide | 50 mg |
| Rosmarinic acid | 50 mg |
| Excipients | 12 mg |

### EXAMPLE 3

Experimental tests certifying the *in vitro* inhibition of the damage from cisplatin to HAI-OC1 cells using the combination of andrographolide and N-acetylcysteine. This experimental model is widely used in the sector of the invention for miming the mechanisms associated with hearing loss.

Specifically, HEI-OC1 cells are cell lines, respectively derived from long-term cultures of the organ of Corti and of the vestibular epithelium, and were used to study drugs for hearing loss, for vestibular disorders and for ototoxicity (Rivolta et al., 2002; Kalinec et al., 2002). Because of its simplicity and ease of handling, it is a recognized model system for the study of mechanisms of action (Kalinec et al., 2003).

### Premises

The ototoxicity of cisplatin is dose and frequency dependant; it presents with hearing loss initially at high frequencies and then progresses towards those of words and is often accompanied by transient or permanent tinnitus (Rademaker-Lakhai et al., 2006).

There is a wide individual variability the causes of which are unknown and can probably be attributed to differences in pharmacokinetics, genetic factors, the metabolic state of the patient (Laurell & Jungnelius, 1990), age, combination with other drugs and already pre-existing poor hearing (Bokemeyer et al., 1998).

In the inner ear cisplatin mainly affects the organ of Corti, the cells of the spiral ganglion and the stria vascularis causing morphological alterations and cell death. In fact, in humans the degeneration of the OHCs (outer hair cells) of the basal turn of the cochlea and in some cases extending to the IHCs (inner hair cells) and to the neurons associated therewith has been highlighted (Rybak et al., 1995, 1999; Evans & Halliwell, 1999; Alam et al., 2000; Huang et al., 2000; Watanabe et al., 2001). Experimentally, cisplatin has been used for decades to mime the mechanisms associated with hearing loss.

The toxicity of cisplatin is greater on the outer hair cells (Laurell & Bagger-Sjoback, 1991, Kopke et al., 1997; Liu et al., 1998; Van Ruijven et al., 2005) and the stria vascularis (Meech et al., 1998), but histological alterations have also been noted at spiral ganglion level in which a significant reduction of the number of cells can be found (Rybak et al., 1995; Cardinaal et al., 2000) as well as a loss of the myelin sheath in type I cells (Van Ruijven et al., 2005).

The toxic effect of cisplatin is expressed through different pathways, one of which implies the formation of DNA adducts that block the progression of the cell cycle (Huang et al., 1995; Kharbanda et al., 1995; Jordan & Carmo-Fonseca, 2000; Kartalou & Essigmann, 2001). In the other pathway cisplatin interacts with the cochlear tissues generating free radicals (ROS; reactive oxygen species) (Clerici et al. 1995, 1996; Kopke et al., 1997; Evans & Halliwell, 1999; Rybak et al., 1999; Feghali et al., 2001; Teranishi et al., 2001) like the superoxide anion (Dehene et al., 2001; Banfi et al., 2004) and nitric oxide (NO) which is formed by the enzyme NOS (Nitric oxide synthase) and is connected with the cochlea both from a physiological and pathological point of view (Feesenden & Schct, 1998). In fact, various authors report that in animals receiving ototoxic doses of cisplatin there was a reduction of glutathione and cellular antioxidants (superoxide dismutase, catalase and glutathione peroxidase and reductase) with an increase in the levels of malonyldialdehyde, an indicator of lipid peroxidation (Ravi et al., 1995; Rybak et al., 2000; Sha et al., 2001). The reduction of antioxidant enzymes at cochlea level can follow from the cisplatin bond with the sulfhydryl groups of the enzymes, the reduction of copper and selenium which are essential for the operation of superoxydismutasis and glutathione peroxidase (DeWoskin & Riviere, 1992), or from the increase of ROS and organic peroxides which inactivate the antioxidant enzymes (Pigeolet et al., 1990), and finally the reduction of glutathione and the cofactor NADPH which are essential for the activity of glutathione peroxidase and reductase (Somani et al., 2001). The inhibition of the antioxidant enzymes due to cisplatin enables the production of ROS and lipid peroxides inside the cochlea; this process causes an increase in the flow of calcium entering the cells with the induction of the apoptotic process both on the hair cells and on the neurons (Clerici et al., 1995; Bowers et al., 2002).

### Materials and Methods

### Cells

Previous studies have reported that Cisplatin (25 mM), an anti-neoplastic agent that has been used in the treatment of tumours, shows toxic effects on the House Ear Institute-Organ Corti 1 cells (HEI-OC1).

HEI-OC1 cells were used, an auditory cell line derived from the organ of Corti in mice. The institution and characterization of the HEI/OC1 cell line, conditionally immortalized, have previously been described by Kalinec et al. (2003). The cells were cultivated in DMEM with a high glucose concentration (Gibco BRL, Grand Island, NY, USA) integrated with 10% fetal bovine serum (Gibco BRL, Grand Island, NY, USA) at 33°C and 5% of CO2 in a humidified atmosphere without antibiotics.

### Cell viability

The cells were inoculated at a density of 8 x 103 cells/well in a plate of 96 wells and left to grow during the night. Then the cells were treated with the formulation containing andrographolides [Andr.] + N-acetylcysteine [NAC] (in the description also referred to as Andr+NAC o OtoP) at the concentrations indicated for 24 hours or with empty medium (control). After gentle washing with PBS, the cells were incubated with MTT solution at 0.5 mg/ml in the dark for 4 hours. DMSO was added in order to dissolve the formazan after removing the supernatant. The absorbance at 570 nm was measured using an ELISA (Multiskan MK3) reader for cell viability.

### Detection of Apoptotic Cell Death

The apoptosis was determined using an ELISA^{PLUS} kit for the detection of cell death (Roche Applied Science, Indianapolis, IN). This photometric immunoenzyme assay provides the quantitative determination of the oligonucleosomes generated by the apoptotic cells. After the treatments, the cells were washed, collected, lysed and centrifuged to remove the nuceli and the supernatants were collected. An aliquot of the supernatant of each sample was incubated with immunoreactants in plates with 96 wells, coated with streptavidin, on a shaker. After three washes with an incubation buffer, the substrate solution was added to each well and the absorbance was read at 405 nm in a microplate reader. The enrichment of the oligonucleosomes released into the cytoplasm was calculated as the absorbance of the cells of the sample/absorbance of the control cells.

### Comparative test

An assay was performed to determine the cell viability through the use of the test MTT (3-(4,5-dimethylthiazol-2-yl)-2,5-diphenyl tetrazolium). The compound MTT was dissolved in PBS at 0.5 mg/ml and sterilized through passage into the Millipore filter (0.22 p.m). This mother liquor was added to the culture wells (1:9) and the plate incubated at 37°C for 1 hour. The enzyme succinate dehydrogenase is active in viable cells, which reduces MTT to formazan crystals giving the solution a blue colour. This reaction was analysed through the spectrophotometric reading of the samples in the plate at a wavelength of 570 nm. This provides an indication of cell viability.

To evaluate the toxicity and survival of the cells to the treatment, the outer hair cells of the organ of Corti were administered the formulation containing Andr. + NAC for 24 hours with three different concentrations (1 µM, 10 µM and 100 µM). Subsequently, the cytoprotective effects of the individual compounds or the Andr. + NAC combination (final concentration 20 µM - Activation Nrf2 observed through Western Blot) were evaluated against the toxicity of cisplatin. The cells were treated for 12 hours with Andr., NAC or Andr. + NAC and subsequently for 6 hours with cisplatin. All the values were expressed as mean ± SEM of at least three cultures. The experiments were repeated at least three times and were performed with the respective controls.

### Statistical Analysis

The data were expressed as mean±standard error of the mean (SEM) of three independent experiments. The statistical significance was determined using the one-way analysis of variance (ANOVA) followed by the Tukey post-hoc test. The value of p < 0.05 was considered statistically significant.

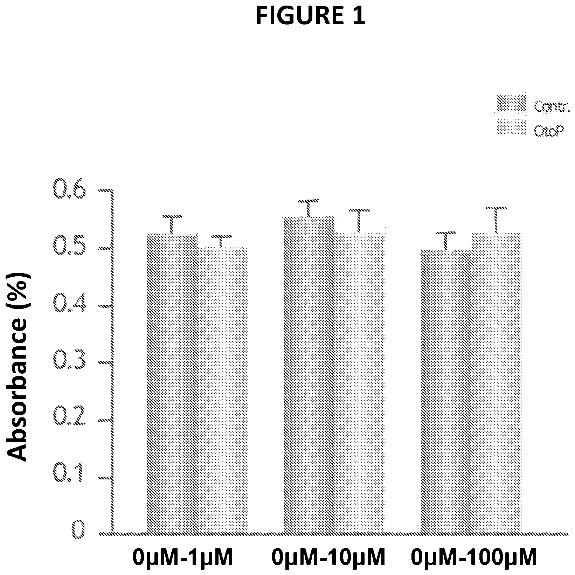

The results of the MTT assay for evaluating any toxicity of the ANDR+NAC combination were illustrated in Figure 1.

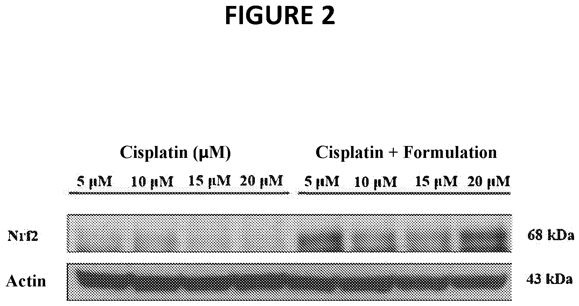

The results of the MTT assay illustrated in Fig. 1 show that the HEI-OC1 cells were preserved well in the cultures treated with Andrographolides [Andr.] + N-Acetylcysteine [NAC] without evidence of selective toxicity at 1, 10 or 100 µM. All the data were expressed as mean±SEM of three independent experiments. Figure 2 highlights how treatment with the ANDR + NAC combination activates the antioxidant transcription factor Nrf2.

In particular, treatment with the formulation Andr + NAC 20 µM strongly increased the expression of the protein transcription factor Nrf2 evaluated through Western Blot assay.

The treatment of HEI-OC1 cells with the formulation (Andr+RosA+NAC) caused a significant increase dependent on the expression time of the protein Nrf2 in the nuclear extracts. The quantification of three independent Western Blots showed that the expression of Nrf2 significantly increased and remained over-regulated up to 12 hours, whereas the levels of the transcription factor actin were stable.

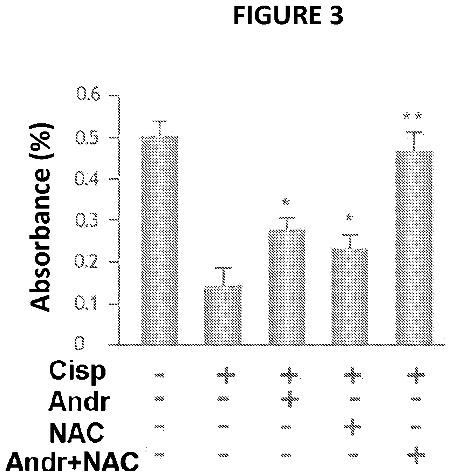

The data illustrated in Figure 3 highlight how treatment with Andr + NAC (OtoP; Andrographolides [Andr.] + N-Acetylcysteine [NAC]) protects the outer hair cells of the organ of Corti from the cytotoxicity induced by cisplatin. The MTT assay was performed to evaluate the cell viability following the cytotoxicity induced by cisplatin and to establish whether treatment with every individual compound and/or their combined administration had cytoprotective effects. The formulation OtoP [20 µM] containing Andr. + NAC was shown to have a significant synergistic cytoprotective effect. The experiments were performed in triplicate.
*, p<0.001 vs Cisp.; **, p<0.0001 vs Cisp.

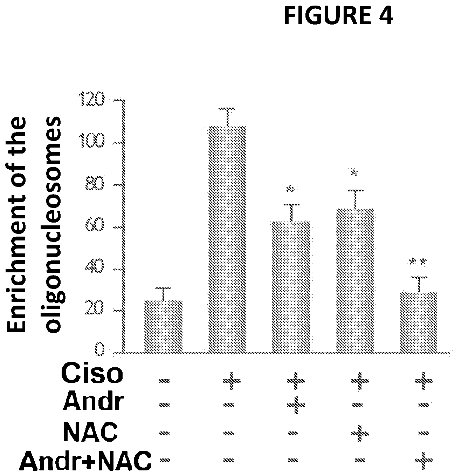

Figure 4 highlights how treatment with the formulation containing Andrographolides [Andr.] + N-acetylcysteine [NAC] significantly reduced the apoptosis rate in cells treated with cisplatin [Cisp.]. The administration of the combination (OtoP; 20 µM) demonstrated a synergistic effect in terms of the reduction of apoptosis. The apoptotic events were measured through the identification in terms of enrichment of oligonucleosomal fragments of DNA, typical structures that characterize apoptosis. The detection method for apoptotic oligonucleosomes envisaged the use of "sandwich enzyme-linked immunosorbent assay". The experiments were performed in triplicate.
*, p<0.001 vs Cisp. **, p<0.0001 vs Cisp.

The co-treatment of Andr. + NAC significantly reduces the apoptosis rate in cells treated with cisplatin. To determine fragments of oligonucleosome of cytoplasmic DNA associated with apoptotic cell death an ELISA kit for the detection of cell death was used. The enrichment of the oligonucleosomes released into the cytoplasm was calculated as the absorbance of the cells of the sample/absorbance of the control cells. The administration of Andr + NAC demonstrated a synergistic effect in terms of the reduction of apoptosis. The results represent the mean ± SEM of three independent experiments. ANOVA followed by the Tukey post-hoc test was performed to determine the significance level.

### References

Alam S. A., Ikeda K., Oshima T., Suzuki M., Kawase T., Kikuchi T. & Takasaka T. (2000). Cisplatin-induced apoptotic cell death in Mongolian gerbil cochlea. Hear. Res.,141: 28-38.
Banfi B., Malgrange B., Knisz J., Steger K., Dubois-Dauphin M., & Crause K. H. (2004). Nox3, a superoxide-generating NADPH oxidase of the inner ear. J. Biol. Chem., 277: 39739-39748.
Bokemeyer C., Berger C. C.,Hartmann J. T. Kollmannsberger C., Schmoll H. J., Kuczyk M. A. & Kanz L. (1998). Analysis of risk factors for cisplatininduced ototoxicity in patients with testicular cancer. Br. J. Cancer, 77: 1355-1362.
Bowers W. J., Chen X., Guo H., Frisina D. R., Federoff H. J. & Frisina R. D. (2002). Neurotrophin-3 transduction attenuates cisplatin spiral ganglion neuron ototoxicity in the cochlea. Mol. Ther., 6: 12-8.
Cardinaal R. M., de Groot J. C., Huizing E. H., Veldman J. E. & Smoorenburg G. F. (2000). Histological effects of co-administration of an ACTH((4-9)) analogue, ORG 2766, on cisplatin ototoxicity in the albino guinea pig. Hear. Res., 144: 157-167.
Clerici W. J., Di Martino D. L. & Prasad M. R. (1995). Direct effect of reactive oxygen species on cochlear outer hair cells. Hear. Res., 84: 30-40.
Clerici W. J., Hensley K., Di Martino D. L. & Butterfield D. A. (1996). Direct detection of ototoxicant-induced reactive oxygen species generation in cochlear explants. Hear Res., 98: 116-124.
Dehne N., Lautermann J., Petrat F., Rauen U. & de Groot H. (2001). Cisplatin ototoxicity: involvement of iron and enhanced formation of superoxide anion radicals. Toxicol. Appl. Pharmacol., 174: 27-34.
DeWoskin R. S. & Riviere J. E. (1992). Cisplatin-induced loss of kidney copper and nephrotoxicity is ameliorated by a single dose of diethyldithiocarbamate but not mesna. Toxicol. Appl. Pharmacol., 112: 182-189.
Evans P. & Halliwell B. (1999). Free radicals and hearing. Cause, consequence, and criteria. Ann. NY Acad. Sci., 884: 19-40.
Feghali J. G., Liu W. & Van de Water T. R. (2001). L-n-acetyl-cysteine protection against cisplatin-induced auditory neuronal and hair cell toxicity. Laryngoscope, 111: 1147-1155.
Fessenden J. D. & Schact J. (1998). The nitric oxide/cyclic GMP pathway: a potential major regulator of cochlear physiology. Hear. Res., 118: 168-76.
Huang H., Zhu L., Reid B. R., Drobny G. P. & Hopkins P. B. (1995). Solution structure of a cisplatin-induced DNA interstrand crosslink. Science, 270: 1842-1845. Huang T., Cheng A.G., Stupak H., Liu W., Kim A., Staecker H., Lefegvre P. P., Malgrange B., Kopke R., Moonen G. & Van de Water T. R. (2000). Oxidative stress induced apoptosis of cochlear sensory cells: otoprotective strategies. Int. J. Dev. Neurosci., 18: 259-270.
Jordan P. & Carmo-Fonseca M. (2000). Molecular mechanisms involved in cisplatin cytotoxicity. Cell. Mol. Life Sci., 57:1229-1235.
Kartalou M. & Essigmann J. M. (2001). Mechanisms of resistance to cisplatin. Mutat. Res., 478: 23-43.
Kharbanda S., Ren R., Pandey P., Shafman T. D., Feller S. M., Weichselbaum, R. R. & Kufe, D. W. (1995). Activation of the c-Abl tyrosine kinase in the stress response to DNA-damaging agents. Nature Lond., 376: 785-788.
Kopke R. D., Liu W., Gabaizadeh R., Jacono A., Feghali J., Spray D., Garcia P., Steinman H., Malgrange B., Ruben R. J., Rybak, L. & Van de Water T. (1997). The use of organotypic cultures of Corti's organ to study the protective effects of antioxidant molecules on cisplatin induced damage of auditory hair cells. Am. J. Otol., 18: 559-571.
Laurell G. & Bagger-Sjoback D. (1991). Degenaration of the organ of Corti following intravenous administration of cisplatin. Acta Otolaryngol (Stockh.), 111: 891- 898. Laurell G. & Jungnelius U. (1990). High-dose cisplatin treatment: hearing loss and plasma concentrations. Laryngoscope, 100: 724- 734.
Liu W., Staecker H., Stupak H., Malgrange B., Lefebvre P. & Van de Water, T. R. (1998). Caspase inhibitors prevent cisplatin-induced apoptosis of auditory sensory cells. NeuroReport, 9: 2609-2614.
Meeche R. P., Campbell K. C., Hughes L. P. & Ribak L. P. (1998). A semiquantitative analysis of the effects of cisplatin on the rat stria vascularis. Hear. Res., 124: 44-59.
Rademarker-Lakhai J. M., Crul M., Zuur L., Baas P., Beijnem J. H., Simis Y. J. W., van Zandwijk N. & Schellens J. H. M. (2006). Relationship between cisplatin administration and the development of ototoxicity. J. Clinic. Oncol., 24 (6): 918-924. Rybak L. P., Husain K., Morris C., Whitworth C. & Somani S. (2000). Effect of protective agents against cisplatin ototoxicity. Am. J. Otol., 21: 513-250.
Rybak L. P., Ravi R. & Somani S. M. (1995). Mechanism of protection by diethydithiocarbamate against cisplatin ototoxicity: antioxidant system. Fundam Appl Toxicol., 26: 293-300.
Rybak L. P., Whitworth C. & Somani S. (1999). Application of antioxidants and other agents to prevent cisplatin ototoxicity. Laryngoscope,109: 1740-1744.
Sha S. H., Taylor R., Forge A. & Schacht J. (2001). Differential vulnerability if basal and apical hair cells is based on intrinsic susceptibility to free radicals. Hearing Research, 155: 1-8.
Somani S. M., Husain K., Jagannathan R. & Rybak L. P. (2001). Amelioration of cisplatin-induced oto- and nephrotoxicity by protective agents. Ann. Neurosci., 8: 101-113.
Teranishi M., Nakashima T. & Wakabayashi T. (2001). Elects of alphatocopherol on cisplatin-induced ototoxicity in guinea pigs. Hear. Res., 151. 61-70.
Van Ruijven M. W. M., de Groot J. C. M. J., Klis S. F. L. & Smoorenburg G. (2005). Cochlear targets of cisplatin: an electrophysiological and morphological timesequence study. Hearing Research, 205: 241- 248.
Watanabe K., Jinnouchi K. & Yagi T. (2001). Detection of singlestranded DNA (ssDNA) in the vestibule of guinea pigs after the application of cisplatinum (CDDP). Anticancer Res., 21: 1135-1138.

## Claims

1. A composition comprising N-acetylcysteine and andrographolide and an edible vehicle for use in the prevention and/or treatment of sensorineural hearing loss of the inner ear.

2. The composition for use according to claim 1, wherein andrographolide is in the form of a plant extract.

3. The composition for use according to any one of claims 1-2, wherein andrographolide is contained in a plant extract of Andrographis paniculata.

4. The composition for use according to claim 1 wherein the sensorineural hearing loss is mild hearing loss with a lowering of the auditory threshold that varies from 20 to 40 dB or moderate hearing loss with a lowering of the auditory threshold that varies from 41 to 65 dB.

5. The composition for use according to any one of claims 1-4, further comprising rosmarinic acid.

6. The composition for use according to any one of claims 1-5, further comprising lutein and/or magnesium.

7. The composition for use according to any one of the claims 1-6, for oral administration.

8. The composition for use according to any one of claims 1-7, in the form of a plant extract, powder, tablet, optionally encapsulated.

9. The composition for use according to any of the claims 1-8, **characterized in** the fact that it is a phyto-drug, a food supplement, a nutraceutical or a medicinal product.

## Patentansprüche

1. Zusammensetzung, umfassend N-Acetylcystein und Andrographolid und einen essbaren Träger zur Verwendung bei der Vorbeugung und/oder Behandlung von sensorineuralem Hörverlust im Innenohr.

2. Zusammensetzung zur Verwendung nach Anspruch 1, wobei Andrographolid in Form eines Pflanzenextrakts vorliegt.

3. Zusammensetzung zur Verwendung nach einem der Ansprüche 1 bis 2, wobei Andrographolid in einem Pflanzenextrakt von Andrographis paniculata enthalten ist.

4. Zusammensetzung zur Verwendung nach Anspruch 1, wobei der sensorineurale Hörverlust ein leichter Hörverlust mit einer Absenkung der Hörschwelle ist, die von 20 bis 40 dB variiert, oder ein mittelschwerer Hörverlust mit einer Absenkung der Hörschwelle, die von 41 bis 65 dB variiert.

5. Zusammensetzung zur Verwendung nach einem der Ansprüche 1 bis 4, ferner umfassend Rosmarinsäure.

6. Zusammensetzung zur Verwendung nach einem der Ansprüche 1 bis 5, ferner umfassend Lutein und/oder Magnesium.

7. Zusammensetzung zur Verwendung nach einem der Ansprüche 1 bis 6, zur oralen Verabreichung.

8. Zusammensetzung zur Verwendung nach einem der Ansprüche 1 bis 7, in Form eines Pflanzenextrakts, Pulvers, einer Tablette, gegebenenfalls verkapselt.

9. Zusammensetzung zur Verwendung nach einem der Ansprüche 1 bis 8, **dadurch gekennzeichnet, dass** sie ein Phytopharmakon, ein Nahrungsergänzungsmittel, ein Nutrazeutikum oder ein Arzneimittel ist.

## Revendications

1. Composition comprenant de la N-acétylcystéine et de l'andrographolide et un véhicule comestible à utiliser pour la prévention et/ou le traitement de la perte auditive neurosensorielle de l'oreille interne.

2. Composition à utiliser selon la revendication 1, dans laquelle l'andrographolide se présente sous la forme d'un extrait végétal.

3. Composition à utiliser selon l'une quelconque des revendications 1 à 2, dans laquelle l'andrographolide est contenu dans un extrait végétal d'Andrographis paniculata.

4. Composition à utiliser selon la revendication 1 dans laquelle la perte auditive neurosensorielle est une perte auditive légère avec un abaissement du seuil auditif qui varie de 20 à 40 dB ou une perte auditive modérée avec un abaissement du seuil auditif qui varie de 41 à 65 dB.

5. Composition à utiliser selon l'une quelconque des revendications 1 à 4, comprenant en outre de l'acide rosmarinique.

6. Composition à utiliser selon l'une quelconque des revendications 1 à 5, comprenant en outre de la lutéine et/ou du magnésium.

7. Composition à utiliser selon l'une quelconque des revendications 1 à 6, pour l'administration orale.

8. Composition à utiliser selon l'une quelconque des revendications 1 à 7, sous forme d'extrait végétal, de poudre, de comprimé, éventuellement encapsulé.

9. Composition à utiliser selon l'une quelconque des revendications 1 à 8, **caractérisée par le fait qu'**il s'agit d'un phytomédicament, d'un complément alimentaire, d'un produit nutraceutique ou d'un produit médicinal.
